(19) Europäisches Patentamt
European Patent Office
Office européen des brevets

(11) **EP 4 686 943 A1**

(12) **EUROPEAN PATENT APPLICATION**

(43) Date of publication:
04.02.2026 Bulletin 2026/06

(21) Application number: 24192139.4

(22) Date of filing: **31.07.2024**

(51) International Patent Classification (IPC):
***G01N 33/543*** (2006.01)   ***G01N 21/552*** (2014.01)

(52) Cooperative Patent Classification (CPC):
**G01N 33/54373; G01N 21/553**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Proteros Biostructures GmbH**
**82152 Martinsried (DE)**

(72) Inventors:
• **Daub, Herwin Alexander**
**81249 Munich (DE)**

• **Aretz, Jonas**
**81475 Munich (DE)**
• **Kröger, Marie**
**85716 Unterschleißheim (DE)**
• **Witte, David Johannes**
**80687 Munich (DE)**
• **Picchianti, Lorenzo**
**33010 Tavagnacco (Udine) (IT)**
• **Nagel, Felix Andreas**
**82131 Gauting (DE)**

(74) Representative: **Maiwald GmbH**
**Elisenhof**
**Elisenstraße 3**
**80335 München (DE)**

(54) **METHOD OF IDENTIFYING ONE OR MORE COMPOUNDS CAPABLE OF BINDING TO A BIOLOGICAL TARGET**

(57)   The present disclosure relates to a sensor-based high-throughput screening method for identifying one or more compounds capable of binding to a biological target or a fragment thereof by high-throughput screening, and a device adapted to carry out said method.

EP 4 686 943 A1

Fig. 2

**Description**

**Technical Field**

The present disclosure relates to a method of identifying one or more compounds capable of binding to a biological target or fragment thereof and a device adapted to carry out said method.

**Background**

Identifying small molecules (such as molecules having a molecular weight in the range of from 300 to 10,000 Da) binding to a biological target of interest, such as a protein involved in the pathology of a disease to be treated, is a major hurdle for successful drug development.

Often, such small molecules are identified by screening large libraries of synthetic compounds or natural products; a method which is also referred to as high throughput screening (HTS).

As such, HTS involves the rapid testing of thousands to millions of chemical compounds for their ability to bind to (and hence modulate) a biological target relevant to a disease. While HTS has revolutionized the drug discovery process by enabling the screening of large compound libraries, it is also a tedious, resource-intensive, time-consuming, expensive, and error-prone task.

Therefore, designing robust assays that accurately reflect the biological target's function or pathway is essential. Developing high-quality assays requires optimization of conditions such as reagent concentrations, incubation times, and detection methods to ensure reliability and reproducibility. For example, specific functional assays can be developed for each target protein or cellular function that is studied. However, development of such functional assays is often costly and difficult, and represents a bottleneck if larger numbers of target proteins are to be studied.

Furthermore, HTS assays can generate "false positives" (compounds that appear to bind to the biological target in an HTS assay but said binding is non-specific) and "false negatives" (active compounds capable of binding to the biological target of interest but that produce a negative result in the assay). While "false positives" may be identified in subsequent confirmatory assays, "false negatives" cannot be identified and are hence easily lost for the future development although they actually have promising properties. Therefore, minimizing false results requires rigorous validation of hits through secondary assays and optimizing screening conditions to reduce assay artifacts.

Besides, also processing and analyzing large volumes of screening data generated from HTS campaigns can be daunting. Efficient data management systems and robust computational algorithms are required to handle data storage, retrieval, analysis, and visualization effectively.

For addressing some of the above issues, surface plasmon resonance (SPR) was introduced as a technique to identify compounds capable of binding to a biological target that is immobilized on a sensor surface. In essence, SPR is based on a change in refractive index upon binding of molecules to the biological target immobilized on a surface. This alteration in the refractive index shifts the resonance angle, which can be measured as a change in the intensity of the reflected light. In other words, binding events between a biological target, such as a protein, and a small molecule can be observed as shifts in the SPR signal. By monitoring said changes in the intensity of the reflected light as a function of angle or time, one can detect and quantify molecular interactions in real-time.

Thereby, the magnitude of the SPR signal shift is proportional to the mass of the molecules bound to the biological target immobilized on the sensor surface; the time course of the SPR signal shift can be used to determine parameters of the binding kinetics, such as association rate constants ($k_a$), dissociation rate constants ($k_d$), and equilibrium dissociation constants ($K_D$).

While SPR is a powerful tool for studying molecular interactions and is extensively used in drug discovery for hit identification, lead optimization, and mechanistic studies, it is not employed in HTS of large compound libraries, because SPR assays typically require longer assay times compared to other HTS techniques such as fluorescence-based assays or luminescence-based assays. This limits the throughput of SPR, making it less suitable for screening large compound libraries containing thousands to millions of compounds. Furthermore, running HTS campaigns using SPR is cost-prohibitive due to the high instrumentation and operating costs associated with SPR technology. In other words, SPR-based screenings cannot cover a large chemical space, i.e. compounds having diverse chemical structures which is essential in HTS: The success of HTS heavily relies on the diversity and quality of the compound library being screened. Compounds with diverse chemical structures increase the likelihood of identifying hits with varied mechanisms of action.

Therefore, SPR is at most used in fragment-based screening methods. Fragment-based screening methods are based on the idea of testing small molecules, i.e. compounds with a molecular weight below about 300 Da. In this manner, a broad chemical space may be covered. Nevertheless, fragments identified through fragment-based screenings typically exhibit weak binding affinities to the biological target of interest, often in the micromolar to millimolar range, which may be difficult to detect.

Furthermore, such fragments lack structural complexity, which can limit their utility as starting points for lead optimization. Developing single fragment hits into drug-like molecules often requires significant chemical modifications, which may introduce challenges related to synthetic feasibility, medicinal chemistry, and property optimization. Besides, fragments may show high unspecific binding to the biological target. Validating the functional significance of fragments and their potential for modulating the biological target can be challenging and may require additional biochemical, biophysical, or cellular assays.

## Summary

In view of the above, there is still a high need for fast, cost-efficient, and sensitive methods for identifying potential lead structures or drug candidates capable of binding to a biological target or fragment thereof by screening large compound libraries, i.e., by high-throughput screening (HTS).

The present invention at least partially aims to overcome the problems associated with current methods of identifying compounds capable of binding to a biological target or fragment thereof by high-throughput screening (HTS).

In a first aspect, the present disclosure relates to a method of identifying one or more compounds capable of binding to a biological target or a fragment thereof by high-throughput screening, said method comprising the steps of:

> a) Providing a compound library comprising at least n compounds, wherein n is the number of compounds comprised in said compound library;
> b) Preparing x pooled samples, wherein x is the number of pooled samples, by mixing each compound of the at least n compounds comprised in the compound library with r further compounds comprised in said compound library, wherein each compound of the at least n compounds is contained in a number y of the x pooled samples, wherein y is at least 2, and r is at least 2;
> c) Providing a sensor surface having the biological target or fragment thereof immobilized on said sensor surface;
> d) Contacting each pooled sample of the x pooled samples with the sensor surface;
> e) For each pooled sample of the x pooled samples, determining a value indicative of a binding of one or more of the r+1 compounds in a pooled sample to the biological target or fragment thereof; and
> f) Identifying the one or more compounds capable of binding to the biological target or fragment thereof by deconvolution analysis of the values determined in step e).

In a second aspect, the present disclosure relates to a device adapted to carry out the method according to the first aspect of the present disclosure.

## Brief description of figures

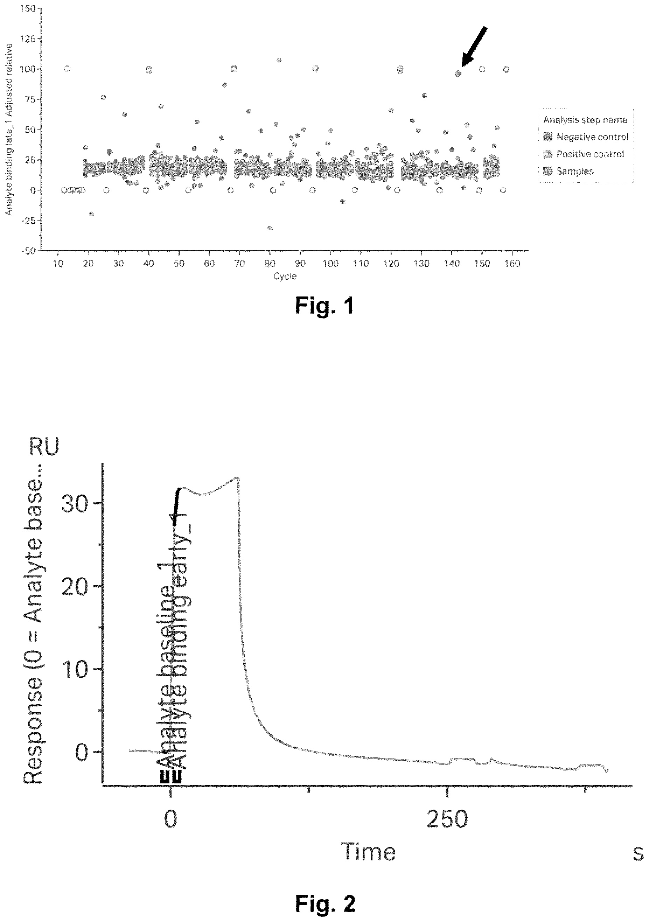

Fig. 1 shows a plot of the maximum response value determined for each of the pooled samples, positive controls, and negative controls of one screening run. Relative maximum binding response normalized to positive control binding is plotted versus injection cycle. Open points at ordinate value equals 100 correspond to positive controls, open points at ordinate value equals zero correspond to negative controls, and solid points represent response values of pooled samples. An arrow highlights the example shown in Fig. 2 in more detail. Plot was created using Biacore Insight Evaluation Software Version 5.0.18.22102, with Software Extension: "Extended Screening".

Fig. 2 shows the time-dependent response curve determined in example 3 for the pooled sample highlighted with an arrow in Fig. 1. The graph was created using Biacore Insight Evaluation Software Version 5.0.18.22102, with Software Extension: "Extended Screening".

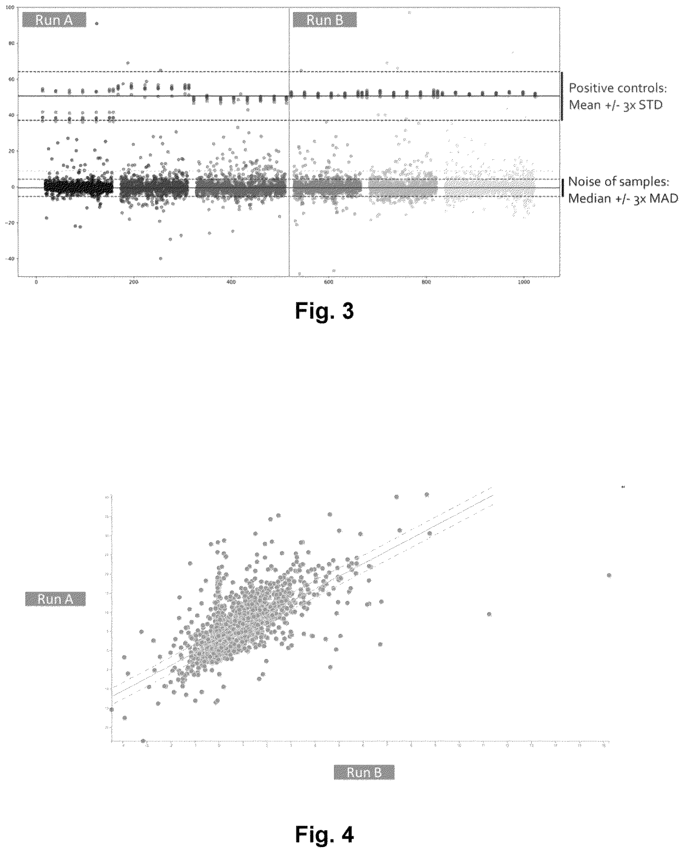

Fig. 3 shows the results obtained from the method according to example 3 of a first run A and a second run B in a side-by-side comparison. Maximum response values were corrected for plate drifts using linear regression (SciPy Version 1.9.3). Data shows corrected maximum response values of each of the pooled samples. Plots were created using Matplotlib Version 3.6.3.

Fig. 4 shows a correlation plot of values indicative of a binding of one or more of the 10 compounds in a pooled sample to the biological target as determined in example 3. Datapoints represent maximum responses values associated with individual compounds of a first run A (ordinate) and a second run B (abszissa). Linear correlation identifies compounds significantly contributing to the maximum response signal of a pooled sample. The plot was exported from visualization tool of web-based database "Collaborative Drug Discovery (CDD) vault".

**Detailed Description**

In a first aspect, the present disclosure relates to a method of identifying one or more compounds capable of binding to a biological target or a fragment thereof by high-throughput screening, said method comprising the steps of:

a) Providing a compound library comprising at least n compounds, wherein n is the number of compounds comprised in said compound library;
b) Preparing x pooled samples, wherein x is the number of pooled samples, by mixing each compound of the at least n compounds comprised in the compound library with r further compounds comprised in said compound library, wherein each compound of the at least n compounds is contained in a number y of the x pooled samples, wherein y is at least 2, and r is at least 2;
c) Providing a sensor surface having the biological target or fragment thereof immobilized on said sensor surface;
d) Contacting each pooled sample of the x pooled samples with the sensor surface;
e) For each pooled sample of the x pooled samples, determining a value indicative of a binding of one or more of the r+1 compounds in a pooled sample to the biological target or fragment thereof; and
f) Identifying the one or more compounds capable of binding to the biological target or fragment thereof by deconvolution analysis of the values determined in step e).

The inventors of the present disclosure have surprisingly found that the throughput (in terms of number of compounds to be screened in a certain time) of a high-throughput screening method using a sensor-based technique for identifying one or more compounds capable of binding to a biological target or fragment thereof can be considerably improved.
In particular, the method of the first aspect of the present disclosure solves a long-felt need for high-throughput methods for scanning a large chemical space (i.e., chemical compounds with a high diversity of their structures) without being limited to fragment-based screenings. To the contrary, compound libraries for classic high-throughput screening, such as commercially available compound libraries, can be screened in a fast and efficient manner in the method according to the present disclosure.
The method according to the first aspect of the present disclosure in principle comprises six steps:

Providing a compound library (step a))

The method according to the first aspect of the present disclosure comprises a first step of providing a compound library comprising at least n compounds, wherein n is the number of compounds comprised in said compound library.
In the context of the present disclosure, it is understood that the compound library comprising at least n compounds is commercially available. In general, the selection of a compound library comprising at least n compounds is not particularly limited. However, it is preferable that the compound library comprises chemical compounds with a large diversity and/or covering a large chemical space.
According to a preferred embodiment of the present disclosure, the compound library is characterized in that n is at least 1,000. In other words, it is a preferred embodiment of the present disclosure that the compound library comprises a least 1,000 different compounds.
It is noted that screening a library comprising 1,000 compounds by classical methods, i.e. using methods without pooling as explained in the following, are already very time consuming and expensive because each of the compounds comprised in a compound library must be contacted with the sensor surface having a biological target or fragment thereof immobilized on said sensor surface separately. Besides the time needed for the actual measurement (including the contact time between the compound and the immobilized target or fragment thereof), sensor-based screening methods also require a certain time for re-equilibrating the sensor after each measurement. Therefore, the method according to the first aspect of the present disclosure is particularly useful for screening large compound libraries and offers a valuable saving in time and cost.
According to a preferred embodiment of the present disclosure, the compound library is characterized in that n is at least 5,000. According to a preferred embodiment of the present disclosure, the compound library is characterized in that n is at least 10,000. According to a preferred embodiment of the present disclosure, the compound library is characterized in that n is at least 20,000. According to a preferred embodiment of the present disclosure, the compound library is characterized in that n is at least 30,000.
It is understood that the size of the compound library is not particularly limited.
According to a preferred embodiment of the present disclosure, the compound library is characterized in that n is 1,000,000 or less. According to another preferred embodiment of the present disclosure, the compound library is characterized on that n is 500,000 or less. According to another preferred embodiment of the present disclosure, the compound library is characterized on that n is 250,000 or less. According to another preferred embodiment of the present disclosure, the compound library is characterized on that n is 100,000 or less.

In a preferred embodiment of the present disclosure, the maximum number of compounds n comprised in the compound library is limited by the pool size (r+1). The larger the pool size, the larger the maximum number of compounds n comprised in a compound library.

According to another preferred embodiment of the present disclosure, the method is characterized in that the ratio of n/(r+1) is 100,000 or less. According to another preferred embodiment of the present disclosure, the method is characterized in that the ratio of n/(r+1) is 50,000 or less. According to a further preferred embodiment of the present disclosure, the method is characterized in that the ratio of n/(r+1) is 30,000 or less. According to a yet further preferred embodiment of the present disclosure, the method is characterized in that the ratio of n/(r+1) is 20,000 or less.

It is understood that the compound library provided in step a) of the method according to the first aspect of the present disclosure is suitable for classical high throughput screening. In other words, the compound library provided in step a) of the method according to the first aspect of the present disclosure comprises only a small proportion of fragments.

As used herein, the term "fragment" refers to a compound having a molecular weight of less than 300 Da.

According to a preferred embodiment of the present disclosure, the compound library comprises at least n compounds, wherein the mean molecular weight of the at least n compounds $M_{W,n}$ is at least 320 Da. According to another preferred embodiment of the present disclosure, the compound library comprises at least n compounds, wherein the mean molecular weight of the at least n compounds $M_{W,n}$ is at least 350 Da. According to a further preferred embodiment of the present disclosure, the compound library comprises at least n compounds, wherein the mean molecular weight of the at least n compounds $M_{W,n}$ is at least 400 Da.

According to a preferred embodiment of the present disclosure, the compound library comprises at least n compounds, wherein the mean molecular weight of the at least n compounds $M_{W,n}$ is in the range of from about 320 Da to about 1,000 Da. According to another preferred embodiment of the present disclosure, the compound library comprises at least n compounds, wherein the mean molecular weight of the at least n compounds $M_{W,n}$ is in the range of from about 350 Da to about 800 Da. According to another preferred embodiment of the present disclosure, the compound library comprises at least n compounds, wherein the mean molecular weight of the at least n compounds $M_{W,n}$ is in the range of from about 400 Da to about 600 Da.

According to another preferred embodiment of the present disclosure, the compound library comprises at least n compounds, wherein at least 75 %(n/n) of the at least n compounds have a molecular weight $M_W$ of 300 Da or more. According to another preferred embodiment of the present disclosure, the compound library comprises at least n compounds, wherein at least 80 %(n/n) of the at least n compounds have a molecular weight $M_W$ of 300 Da or more. According to another preferred embodiment of the present disclosure, the compound library comprises at least n compounds, wherein at least 85 %(n/n) of the at least n compounds have a molecular weight $M_W$ of 300 Da or more. According to a further preferred embodiment of the present disclosure, the compound library comprises at least n compounds, wherein at least 90 %(n/n) of the at least n compounds have a molecular weight $M_W$ of 300 Da or more. According to a yet further preferred embodiment of the present disclosure, the compound library comprises at least n compounds, wherein at least 95 %(n/n) of the at least n compounds have a molecular weight $M_W$ of 300 Da or more. According to an even further preferred embodiment of the present disclosure, the compound library comprises at least n compounds, wherein at least 98 %(n/n) of the at least n compounds have a molecular weight $M_W$ of 300 Da or more.

In other words, the inventors of the present disclosure have surprisingly found that the method of the present disclosure is particularly useful and efficient for screening compound libraries that contain a low number of fragments or even no fragments. Thus, the method of the present disclosure is not limited to fragment-based screening method, but satisfies the long-felt need for sensor-based high-throughput screenings of compound libraries containing lead structure candidates with high molecular weight and complex chemical structure.

Preparing x pooled samples (step b))

The method according to the first aspect of the present disclosure comprises a second step of preparing x pooled samples, wherein x is the number of pooled samples, by mixing each compound of the at least n compounds comprised in the compound library with r further compounds comprised in said compound library, wherein each compound of the at least n compounds is contained in a number y of the x pooled samples, wherein y is at least 2, and r is at least 2.

The inventors of the present disclosure have surprisingly found that large compound libraries as defined hereinabove (see description of step a)) can be screened for compounds capable of binding to a biological target or fragment thereof in a fast and efficient manner with high accuracy. Thereby, the number of samples to be measured is considerably reduced by pooling each of the at least n compounds comprised in the compound library with two or more further compounds from said library. Thus, the number of samples to be tested is reduced significantly, which also results in significant time savings when assaying a large number of compounds.

Each of the pooled samples may be prepared by mixing each of the at least n compounds comprised in the compound library provided in step a) with r further compounds from said compound library, e.g. as shown in example 2.

Said mixing may be performed by any method known to the person skilled in the art. For example, solutions of each of the at least n compounds comprised in the compound library provided in step a) in a biocompatible solvent, such as DMSO, water or a mixture of DMSO and water, may be prepared in a separate wells of commercially available well plates, and an automated liquid handling system may be configured to select solutions of each of the at least n compounds from their respective wells and mix them in a new well. According to a preferred embodiment of the present disclosure, the automated liquid handling system is configured for nL transfers. According to another preferred embodiment of the present disclosure, mixing is performed by acoustic dispensing. According to another preferred embodiment of the present disclosure, mixing is performed by a dipping robot.

According to a preferred embodiment of the present disclosure, preparing the x pooled samples may be performed by creating permanent pooled samples.

According to a preferred embodiment of the present disclosure, preparing the x pooled plates may be performed by ad-hoc creation of pooled samples. In the context of the present disclosure, the term "ad-hoc creation of pooled samples" refers to the spontaneous or on-the-fly preparation of pooled samples based on specific experimental needs or circumstances. Ad-hoc creation of pooled samples allows researchers to dynamically adapt their pooling strategy in real-time based on emerging insights, experimental outcomes, or logistical considerations.

For the method according to the first aspect of the present disclosure, it is essential that each compound of the at least n compounds comprised in the compound library provided in step a) is contained in a number y of the x pooled samples, wherein y is at least 2. If y is only one, deconvolution of the values indicative of a binding of one or more compounds contained in a pooled sample to a biological target or fragment thereof is not possible. In other words, each of the at least n compounds comprised in the compound library provided in step a) is contained in at least two different pooled samples.

According to a preferred embodiment of the present disclosure, y is at least 2. According to another preferred embodiment of the present disclosure, y is at least 3.

The inventors of the present disclosure have surprisingly found that a compound capable of binding to a biological target or fragment thereof can be unambiguously identified by the method of the first aspect of the present disclosure when said compound present in two or three or more pooled samples.

According to a preferred embodiment of the present disclosure, y is 7 or less. According to another preferred embodiment of the present disclosure, y is 5 or less. According to a further preferred embodiment of the present disclosure, y is 4 or less. At the same time, it is understood that y is at least 2.

The inventors of the present disclosure have surprisingly found that a compound capable of binding to a biological target or fragment thereof can be unambiguously identified by the method of the first aspect of the present disclosure when said compound present in seven, five, four, or even less (but at least two) pooled samples. The lower the number y, the lower the number of pooled samples x that need to be tested.

According to a preferred embodiment of the present disclosure, y is 2. According to another preferred embodiment of the present disclosure, y is 5. According to a further preferred embodiment of the present disclosure, y is 4. According to a yet further embodiment of the present disclosure, y is 3. According to an even further preferred embodiment of the present disclosure, y is 2 or 3.

According to a preferred embodiment of the present disclosure, y is in the range of from 2 to 7. According to another preferred embodiment of the present disclosure, y is in the range of from 2 to 5. According to a further preferred embodiment of the present disclosure, y is in the range of from 3 to 5.

For the method according to the first aspect of the present disclosure, the step of preparing the x pooled samples includes mixing each compound of the at least n compounds comprised in the compound library provided in step a) with r further compounds comprised in said compound library. The parameter "r" refers to the number of further compounds that are mixed with each of the n compounds comprised in the compound library comprising at least n compounds. According to the first aspect of the present disclosure, r is at least 2. Hence, the number "r+1" is representative of the number of compounds contained in a pooled sample. In other words, each of the x pooled samples contains at least 3 structurally different compounds selected from the at least n compounds comprised in the compound library provided in step a).

It is understood that each of the n compounds comprised in the compound library must be present in at least two of the x pooled samples. In this context, it is understood that each of the x pooled samples contains r+1 of the n compounds comprised in the compound library provided in step a). The inventors of the present disclosure have surprisingly found that mixing each of the n compounds comprised in the compound library provided in step a) of the method according to the present disclosure with r further compounds not only reduces the number of samples to be screened in a method of high-throughput screening, but at the same time provides for a reliable identification of the one or more compounds capable of binding to a biological target or fragment thereof as shown in example 4.

According to a preferred embodiment of the present disclosure, r is at least 6. According to another preferred embodiment of the present disclosure, r is at least 9. According to another preferred embodiment of the present disclosure, r is at least 12. According to a further preferred embodiment of the present disclosure, r is at least 15.

In other words, according to a preferred embodiment of the present disclosure, each of the x pooled samples contains at least 7 different compounds ($r \geq 6$) selected from the at least n compounds comprised in the compound library provided in step a). According to another preferred embodiment of the present disclosure, each of the x pooled samples contains at least 10 different compounds ($r \geq 9$) selected from the at least n compounds comprised in the compound library provided in step a). According to another preferred embodiment of the present disclosure, each of the x pooled samples contains at least 13 different compounds ($r \geq 12$) selected from the at least n compounds comprised in the compound library provided in step a). According to a further preferred embodiment of the present disclosure, each of the x pooled samples contains at least 16 different compounds ($r \geq 15$) selected from the at least n compounds comprised in the compound library provided in step a).

According to a preferred embodiment of the present disclosure, r is 200 or less. According to another preferred embodiment of the present disclosure, r is 150 or less. According to another preferred embodiment of the present disclosure, r is 100 or less. According to another preferred embodiment of the present disclosure, r is 80 or less. According to a further preferred embodiment of the present disclosure, r is 60 or less. According to a yet further preferred embodiment of the present disclosure, r is 40 or less.

In other words, according to a preferred embodiment of the present disclosure, each of the x pooled samples contains 201 or less different compounds ($r \leq 200$) selected from the at least n compounds comprised in the compound library provided in step a). According to another preferred embodiment of the present disclosure, each of the x pooled samples contains 151 or less different compounds ($r \leq 150$) selected from the at least n compounds comprised in the compound library provided in step a). According to another preferred embodiment of the present disclosure, each of the x pooled samples contains 101 or less different compounds ($r \leq 100$) selected from the at least n compounds comprised in the compound library provided in step a). According to another preferred embodiment of the present disclosure, each of the x pooled samples contains 81 or less different compounds ($r \leq 80$) selected from the at least n compounds comprised in the compound library provided in step a). According to a further preferred embodiment of the present disclosure, each of the x pooled samples contains 61 or less different compounds ($r \leq 60$) selected from the at least n compounds comprised in the compound library provided in step a). According to a yet further preferred embodiment of the present disclosure, each of the x pooled samples contains 41 or less different compounds ($r \leq 40$) selected from the at least n compounds comprised in the compound library provided in step a).

According to a preferred embodiment of the present disclosure, r is in the range of from 2 to 200. According to another preferred embodiment of the present disclosure, r is in the range of from 6 to 200. According to another preferred embodiment of the present disclosure, r is in the range of from 9 to 150. According to another preferred embodiment of the present disclosure, r is in the range of from 9 to 100. According to another preferred embodiment of the present disclosure, r is in the range of from 9 to 80. According to another preferred embodiment of the present disclosure, r is in the range of from 9 to 60. According to another preferred embodiment of the present disclosure, r is in the range of from 9 to 40. According to a further preferred embodiment of the present disclosure, r is in the range of from 9 to 24. According to a yet further preferred embodiment of the present disclosure, r is in the range of from 9 to 19.

According to another preferred embodiment of the present disclosure, r is in the range of from 12 to 80. According to another preferred embodiment of the present disclosure, r is in the range of from 12 to 60. According to another preferred embodiment of the present disclosure, r is in the range of from 12 to 40. According to a further preferred embodiment of the present disclosure, r is in the range of from 15 to 40.

It is understood that any method known to the skilled person may be used for selecting the r+1 compounds contained in each of the pooled samples. The act of selecting the r+1 compounds contained in each of the pooled samples is also referred to as "creating the pooled samples" or "pooling strategy".

According to a preferred embodiment of the present disclosure, step b) is performed by using an orthogonal pooling strategy.

An orthogonal pooling strategy is characterized in that the at least n compounds comprised in the compound library are grouped into pooled samples in a systematic and structured manner. Thereby, it is made sure that each of the at least n compounds comprised in the compound library is present in two of the x pooled samples.

If each of the at least n compounds comprised in the compound library is contained in two of the x pooled samples,

$$X = \frac{2 \cdot n}{(r+1)}$$

the number of pooled samples x may be calculated as $\phantom{X}$.

It is clear from the above equation that the number of samples that need to be measured for screening the entire compound library is considerably reduced compared to testing each of the at least n compounds comprised in the compound library ("one compound - one well"), even when relatively small pools are prepared, i.e. pools comprising only three (r+1 with r being 2) different compounds of the at least n compounds comprised in the compound library.

According to a preferred embodiment of the present disclosure, step b) is performed by using an orthogonal pooling strategy; and x is $\frac{2 \cdot n}{(r+1)}$.

By increasing the pool size, i.e. by increasing r as set out hereinabove, the number of pooled samples that needs to be subjected to the subsequent steps can be further reduced.

According to another preferred embodiment of the present disclosure, step b) is performed by using an adaptive pooling strategy.

An adaptive pooling strategy is characterized in that the pooling is performed in stages. In a first stage, the at least n compounds comprised in the compound library are grouped into pooled samples in a systematic and structured manner. Said pooled samples are subjected to the assay, i.e. steps d) to e) as disclosed herein, in a first screening. Based on the information gained from the first screening, further pooled samples containing other combinations of the at least n compounds comprised in the compound library are designed. Said pooled samples are then subjected to the assay, i.e. steps d) to e) as disclosed herein, in a second screening.

According to another preferred embodiment of the present disclosure, step b) is performed by using a non-adaptive pooling strategy.

A non-adaptive pooling strategy is characterized in that the at least n compounds comprised in the compound library are grouped into pooled samples in a systematic and structured manner. Thereby, it is made sure that each of the at least n compounds comprised in the compound library is present in a number y of the x pooled samples.

By also increasing y as described above, that is the number of pooled samples containing each of the at least n compounds comprised in the compound library, the one or more compound capable of binding to a biological target or fragment thereof can be identified with high accuracy. Furthermore, the risk of "false negative" results, that are compounds that do apparently not bind to the biological target of interest or fragment thereof and/or that are not classified as binding to the biological target of interest or fragment thereof based on the value indicative of binding determined in step e) measured in a single assay, is mitigated, because each of the compounds is tested several times. False negative results are particularly critical to a high-throughput screening because false negatives are excluded from further development of drug candidates although they - in reality - have promising properties.

According to a further preferred embodiment of the present disclosure, step b) is performed by using a non-adaptive pooling strategy; and x is $\frac{y \cdot n}{(r+1)}$.

The inventors of the present disclosure have surprisingly found that it is possible to identify one or more compounds capable of binding to a biological target or fragment thereof from large pools (that are pooled samples with a high value r) with high accuracy.

Therefore, according to a preferred embodiment of the present disclosure, step b) is performed by using an adaptive or non-adaptive pooling strategy, and x is $\frac{y \cdot n}{(r+1)}$.

According to a preferred embodiment of the present disclosure, x is $\frac{y \cdot n}{(r+1)}$, wherein r is at least 20 and y is in the range of from 3 to 5. According to another preferred embodiment of the present disclosure, x is $\frac{y \cdot n}{(r+1)}$, wherein r is at least 20 and y is 3, or 4, or 5. According to another preferred embodiment of the present disclosure, x is $\frac{y \cdot n}{(r+1)}$, wherein r is in the range of from 20 to 200 and y is in the range of from 3 to 5. According to a further preferred embodiment of the present disclosure, x is $\frac{y \cdot n}{(r+1)}$, wherein r is in the range of from 20 to 100 and y is in the range of from 3 to 5.

The inventors of the present disclosure have surprisingly found that the time needed to screen large compound libraries comprising at least n compounds as disclosed herein is significantly reduced by applying the above pooling strategy, wherein x is $\frac{y \cdot n}{(r+1)}$.

For example, one or more compounds capable of binding to a biological target or fragment thereof are identified from pooled samples containing 100 different compounds (r+1 is 100) selected from the at least n compounds comprised in the compound library as disclosed herein in a time-efficient and accurate manner, if each of the at least n compounds comprised in said compound library is contained in only three to five of the pooled samples (y is 3 to 5, such as 3, or 4, or 5). Therefore, the number of samples to be tested in a high-throughput screening method is significantly reduced (with y being 3) by 97 % when compared to testing each of the at least n compounds comprised in the compound library in a "one compound - one well" strategy.

Hence, the inventors of the present disclosure have surprisingly found that also large compound libraries can be screened in a cost and time efficient manner using a sensor-based assay for high-throughput screening.

<u>Providing a sensor surface having the biological target or fragment thereof immobilized on said sensor surface (step c))</u>

The method according to the first aspect of the present disclosure comprises a third step of providing a sensor surface having the biological target or fragment thereof immobilized on said sensor surface.

Hence, it is understood that the present disclosure relates to a method of identifying one or more compounds capable of binding to a biological target or fragment thereof by high-throughput screening using a sensor-based assay, but not an "in-solution" assay, such as fluorescence-based assays and/or biochemical assays.

While sensor-based assays have been considered valuable tools in hit validation, lead optimization, and mechanism of action studies, sensor-based assays have not been used as a primary method for high-throughput screening.

The inventors of the present disclosure have now surprisingly found that sensor-based assays are also useful in primary methods for high-throughput screening, i.e. for identifying one or more compounds capable of binding to a biological target or fragment thereof from a large compound library.

In that, the present disclosure at least partially overcomes previous constraints of sensor-based assays in high-throughput screening, that are, in particular, limitations of throughput in terms of compounds that can be tested in a specific duration of time.

The sensor surface may be part of any sensor that is suitable for sensor-based binding assays, such as surface-plasmon-resonance (SPR).

Such sensors are typically based on label-free techniques, detecting a change in a property of the sensor surface, such as change in mass, refractive index, or thickness of the immobilized layer. Typical sensor detection techniques include, but are not limited to, mass detection methods, such as optical, thermo-optical and piezoelectric or acoustic wave methods (including e.g. surface acoustic wave (SAW) and quartz crystal microbalance (QCM) methods), and electrochemical methods, such as potentiometric, conductometric, amperometric and capacitance/impedance methods. With regard to optical detection methods, representative methods include those that detect mass surface concentration, such as reflection-optical methods, including both external and internal reflection methods, which are angle, wavelength, polarization, or phase resolved, for example evanescent wave ellipsometry and evanescent wave spectroscopy (EWS, or Internal Reflection Spectroscopy), both of which may include evanescent field enhancement via surface plasmon resonance (SPR), Brewster angle refractometry, bio-layer interferometry (BLI), grating-coupled interferometry (GCI), critical angle refractometry, frustrated total reflection (FTR), scattered total internal reflection (STIR) (which may include scatter enhancing labels), optical wave guide sensors; external reflection imaging, evanescent wave-based imaging such as critical angle resolved imaging, Brewster angle resolved imaging, SPR-angle resolved imaging, and the like. Further, photometric and imaging/microscopy methods, "per se" or combined with reflection methods, based on for example surface enhanced Raman spectroscopy (SERS), surface enhanced resonance Raman spectroscopy (SERRS), evanescent wave fluorescence (TIRF) and phosphorescence may be mentioned, as well as waveguide interferometers, waveguide leaky mode spectroscopy, reflective interference spectroscopy (RIfS), transmission interferometry, holographic spectroscopy, and atomic force microscopy (AFR).

According to a preferred embodiment of the present disclosure, the sensor surface is part of a sensor for surface plasmon resonance (SPR). SPR is a technique known to the skilled person. Sensors for SPR permit monitoring of surface binding interaction between a biological target or fragment thereof that is immobilized on the sensor surface and one or more compounds capable of binding to said biological target or fragment thereof in real time. Sensors for SPR are commercially available.

Typical biological targets or fragments thereof that can be immobilized to the sensor surface include, but are not limited to, proteins and glycoproteins (e.g., antibodies or fragments thereof, affibodies, or aptamers), lipids, carbohydrates, enzymes, receptors, antigens, haptens, peptides, ribonucleic acids, ribozymes, or fragments thereof.

According to a preferred embodiment of the present disclosure, the biological target is an enzyme. According to another preferred embodiment of the present disclosure, the fragment of the biological target is a fragment of an enzyme. According to another preferred embodiment, the biological target is a receptor. According to another preferred embodiment of the present disclosure, the fragment of the biological target is a fragment of a receptor.

The biological target or fragment thereof may be immobilized on the sensor surface by any method known to the person skilled in the art.

In a preferred embodiment of the present disclosure, the biological target or fragment thereof is immobilized on the sensor surface by streptavidin-mediated immobilization. In another preferred embodiment of the present disclosure, the biological target or fragment thereof is immobilized on the sensor surface by NTA-amine immobilization. In another preferred embodiment of the present disclosure, the biological target or fragment thereof is immobilized on the sensor surface by direct immobilization, e.g. using a coupling agent such as EDC and/or NHS. Generally, it is understood that the biological target or fragment thereof is immobilized on the sensor surface by any suitable method known to the person skilled in the art.

Contacting each pooled sample with the sensor surface (step d))

The method according to the first aspect of the present disclosure comprises a fourth step of contacting each pooled sample of the x pooled samples with the sensor surface (step d)). It is understood that the sensor surface referred to in step d) has the biological target or fragment thereof immobilized on said sensor surface.

The step of contacting each pooled sample of the x pooled samples with the sensor surface (step d)) may be performed by any method know to the skilled person that allows for a binding of the one or more compounds to the biological target or fragment thereof immobilized on a sensor surface as provided in step c) above.

To improve the throughput of the method, it is preferable that the step of contacting each pooled sample of the x pooled samples with the sensor surface having the biological target or fragment thereof immobilized thereto is performed by flowing the sample over the sensor surface. Subsequently, a washing step may be performed to remove compounds bound to the sensor surface and/or the biological target or fragment thereof immobilized to said sensor surface.

According to a preferred embodiment of the present disclosure, the step of contacting each of the pooled samples of the x pooled samples with the sensor surface having the biological target or fragment thereof immobilized to said sensor surface is performed in a setup of a sensor-based screening method, such as a setup of a sensor based fragment screening.

According to a further preferred embodiment of the present disclosure, step d) is performed by subsequently flowing each of the pooled samples of the x pooled samples over the sensor surface for a contact time $t_c$. According to another further preferred embodiment of the present disclosure, step d) further comprises a step of flowing a buffer over the sensor surface for a washing time $t_w$.

According to a preferred embodiment of the present disclosure, the contact time $t_c$ is in the range of from 30 s to 120 s. According to a preferred embodiment of the present disclosure, the contact time $t_c$ is in the range of from 45 s to 90 s. According to a further preferred embodiment of the present disclosure, the contact time $t_c$ is in the range of from 50 s to 70 s. According to a further preferred embodiment of the present disclosure, the contact time $t_c$ is about 60 s. The inventors of the present disclosure have surprisingly found that a contact time $t_c$ is sufficient to identify all pooled samples containing one or more compounds capable of binding to the biological target or fragment thereof. In other words, a value indicative of binding of one or more of the r+1 compounds contained in a pooled sample to the biological target or fragment thereof as disclosed herein can be reliably determined for each pooled sample of the x pooled samples within said contact time $t_c$. On the other hand, if the contact time $t_c$ exceeds 120 s, the quality of the screening does not further improve but the time consumption is increased.

According to a preferred embodiment of the present disclosure, the washing time $t_w$ is in the range of from 120 s to 300 s. According to another preferred embodiment of the present disclosure, the washing time $t_w$ is in the range of from 120 s to 240 s. According to a further preferred embodiment of the present disclosure, the washing time $t_w$ is in the range of from 150 s to 210 s. According to a yet further preferred embodiment of the present disclosure, the washing time $t_w$ is about 180 s.

It is understood that both the contact time $t_c$ as well as the washing time $t_w$ contribute to the time that is needed for testing a compound using a sensor-based assay, such as SPR. Therefore, it is immediately apparent that the method according to the present disclosure requires considerably less time for screening large compound libraries by reducing the number of samples to be tested.

Determining a value indicative of a binding (step e))

The method according to the first aspect of the present disclosure comprises a fifth step of determining a value indicative of a binding of one or more of the r+1 compounds contained in a pooled sample to the biological target or fragment thereof for each pooled sample of the x pooled samples.

It is understood that the value indicative of a binding of one or more of the r+1 compounds contained in a pooled sample to the biological target or fragment thereof for each pooled sample of the x pooled samples is obtained by a sensor-based method, which studies the molecular interactions and presents the results in real time. In other words, sensor-based assay permit monitoring of binding interactions between one or more of the r+1 compounds contained in a pooled sample to the biological target or fragment thereof immobilized on the sensor surface in real time.

It is understood that said value indicative of a binding of one or more of the r+1 compounds contained in a pooled sample to the biological target or fragment thereof represents a change in a property of the sensor surface, such as change in mass, refractive index, or thickness of the immobilized layer.

In other words, the value indicative of a binding of one or more of the r+1 compounds contained in a pooled sample to the biological target or fragment thereof depends on the type of detector provided in step c). Methods of determining a value indicative of a binding of one or more of the r+1 compounds contained in a pooled sample to the biological target or fragment thereof for each pooled sample of the x pooled samples for each of the sensors provided in step c) are known to the skilled person.

According to a preferred embodiment of the present disclosure, step e) is performed using any one of the sensor detection techniques as disclosed herein or a combination thereof. According to a further preferred embodiment of the present disclosure, step e) is performed using SPR.

According to another preferred embodiment, step e) comprises determining a maximum response value. As used herein, the term "maximum response value" refers to the value indicative of a binding of one or more of the r+1 compounds contained in a pooled sample to the biological target or fragment thereof immobilized on the sensor surface that is measured upon saturation of the binding of said one or more of the r+1 compounds contained in a pooled sample to said biological target or fragment thereof. In other words, the "maximum response value" refers to the highest value indicative of binding of one or more of the r+1 compounds contained in a pooled sample to the biological target or fragment thereof that is determined during contacting a pooled sample with the sensor surface (step d)).

Said maximum response value may be used in step f) of the method of the first aspect of the present disclosure.

For example, said maximum response values may be visualized as shown in Fig. 1 and/or Fig. 3 of the present disclosure.

According to another preferred embodiment, step e) comprises determining a time-dependent response curve. As used herein, the term "time-dependent response curve" refers to the curve obtained by plotting several values indicative of a binding of one or more of the r+1 compounds contained in a pooled sample to the biological target or fragment thereof immobilized on the sensor against the time, wherein each of said several values is determined at a different time point during step d). In other words, the "time-dependent response curve" refers to development of the value indicative of binding of one or more of the r+1 compounds contained in a pooled sample to the biological target or fragment thereof during step d). Hence, the "time-dependent response curve" is representative of the kinetics of binding of one or more of the r+1 compounds contained in a pooled sample to the biological target or fragment thereof.

Said time-dependent response curve may be used in step f) of the method of the first aspect of the present disclosure and further improves the accuracy of identifying the one or more compounds capable of binding to the biological target or fragment thereof.

An example of such time-dependent response curve is shown in Fig. 2 of the present disclosure.

According to a further preferred embodiment of the present disclosure, step e) comprises determining a maximum response value and a time-dependent response curve. In this manner, the accuracy of identifying the one or more compounds capable of binding to the biological target or fragment thereof in step f) can be further increased.

It is understood that the value indicative of binding of one or more of the r+1 compounds contained in a pooled sample to the biological target or fragment thereof for each pooled sample of the x pooled samples depends on the sensor technology.

According to a preferred embodiment of the present disclosure, the value indicative of binding of one or more of the r+1 compounds contained in a pooled sample to the biological target or fragment thereof for each pooled sample of the x pooled samples is a change in refractive index, such as the difference between the refractive index determined by the sensor in the absence of a pooled sample and the refractive index determined by the sensor during step d).

According to another preferred embodiment of the present disclosure, the value indicative of binding of one or more of the r+1 compounds contained in a pooled sample to the biological target or fragment thereof for each pooled sample of the x pooled samples is a thickness, such as the difference between the thickness of a layer immobilized on the sensor surface determined by the sensor in the absence of a pooled sample and the thickness of the layer immobilized on said sensor surface determined by the sensor during step d).

According to another preferred embodiment of the present disclosure, the value indicative of binding of one or more of the r+1 compounds contained in a pooled sample to the biological target or fragment thereof for each pooled sample of the x pooled samples is a mass, in particular the difference between the mass of a layer immobilized on the surface of a sensor surface determined by the sensor in the absence of a pooled sample and the mass of the layer immobilized on said sensor surface determined by the sensor during step d).

According to another preferred embodiment of the present disclosure, the value indicative of binding of one or more of the r+1 compounds contained in a pooled sample to the biological target or fragment thereof for each pooled sample of the x pooled samples is normalized.

For example, in SPR, an increase of the mass close to the sensor surface is determined when one or more compounds bind to the biological target or fragment thereof immobilized on said sensor surface. Thereby, an expected mass increase $m_{expected}$ can be calculated. The actual mass increase $m_{actual}$ is determined in step e). The ratio of

$$\frac{m_{actual}}{m_{expected}}$$

can be expressed in % (RU).

identifying the one or more compounds capable of binding to the biological target or fragment thereof (step f))

The method according to the first aspect of the present disclosure comprises a sixth step of identifying the one or more compounds capable of binding to the biological target or fragment thereof by deconvolution analysis of the values determined in step e).

It is understood that in step f) of the method according to the first aspect of the present disclosure, the values indicative of binding of one or more of the r+1 compounds contained in a pooled sample to the biological target or fragment thereof for each pooled sample of the x pooled samples determined in step e) are used to identify the one or more compounds capable of binding to the biological target or fragment thereof. This step is performed by deconvolution analysis, e.g. as shown in example 4.

In other words, in step f), the values indicative of binding of one or more of the r+1 compounds contained in a pooled sample to the biological target or fragment thereof for each pooled sample of the x pooled samples are assigned to a single compound comprised in the compound library provide in step a).

According to a preferred embodiment of the present disclosure, step f) comprises a step f1) of identifying a plurality of hitpools each containing at least one compound capable of binding to the biological target or fragment thereof.

According to a preferred embodiment of the present disclosure, step f1) is performed by determining a median value from the values for each pooled sample of the x pooled samples determined in step e); and determining a median absolute deviation (MAD) from the values for each pooled sample of the x pooled samples determined in step e); wherein a hitpool is each pooled sample of the x pooled samples having a response value of at least the median response value plus w-times the median absolute deviation (MAD), wherein w is at least 2.

According to a preferred embodiment, w is selected based on the quality of the screening. It is understood that w is selected in a way that the method identifies an appropriate number of compounds capable of binding to the biological target or fragment thereof. According to another preferred embodiment of the present disclosure, w is at least 3. According to another preferred embodiment of the present disclosure, w is in the range of from 2 to 5. According to another preferred embodiment of the present disclosure, w is in the range of from 2.5 to 4.5. According to another preferred embodiment of the present disclosure, w is in the range of from 3.0 to 4.0.

According to another preferred embodiment of the present disclosure, step f) is characterized in that the compound capable of binding to a biological target or a fragment thereof is identified if at least 75 % of the pooled samples containing said compound are hitpools. According to another preferred embodiment of the present disclosure, step f) is characterized in that the compound capable of binding to a biological target or a fragment thereof is identified if at least 80 % of the pooled samples containing said compound are hitpools. According to another preferred embodiment of the present disclosure, step f) is characterized in that the compound capable of binding to a biological target or a fragment thereof is identified if at least 90 % of the pooled samples containing said compound are hitpools.

According to another preferred embodiment of the present disclosure, step f) comprises a step f2) of assigning the value indicative of binding of one or more of the r+1 compounds contained in a hitpool determined in step e) to each of the r+1 compounds contained in said hitpool. Subsequently, a compound is identified as capable of binding to the biological target or fragment thereof, if said compound shows about the same value indicative of binding in at least two hitpools.

According to a preferred embodiment of the present disclosure, compound shows about the same value indicative of binding in at least two hitpools if the values indicative of binding differ by 10 % or less.

According to another preferred embodiment of the present disclosure, step f) comprises using a correlation plot, e.g. as shown in Fig. 4. In such embodiment, each of the r+1 compounds contained in each of the x pooled samples is assigned with the maximum response value measured for the respective pooled sample. It is understood that at this point of step f), said assignment is only partially correct. Then, the assigned maximum response value (ordinate) of each of the compounds determined in a first run (that includes each of the at least n compounds contained in a compound library in one pooled sample) is plotted against the compound number (abzisse). In the same way, also the assigned maximum response value of each of the compounds (abzisse) determined in a second run (that includes each of the at least n compounds contained in a compound library in one pooled sample that is different from the first one) is plotted against the compound number (ordinate). Linear correlation of such graph identifies compounds significantly contributing to pool response signal.

According to another preferred embodiment of the present disclosure, step f) comprises a step f3) of comparing the time-dependent response curves determined in step e). According to another preferred embodiment of the present disclosure, step f) further comprises a step f3) of comparing the time-dependent response curves measured for two hitpools containing the same compound.

Accordingly, a compound is identified as a compound capable of binding to the biological target or fragment thereof, if the time-dependent response curves of at least two hitpools containing said compound are similar.

According to a further preferred embodiment of the present disclosure, f3) is based on a Pearson's correlation, or absolute comparison of reference points in said time-dependent response curves, or relative comparison of reference points in said time-dependent response curves.

According to a further preferred embodiment of the present disclosure, step f) comprises step f1), and step f2) and/or step f3).

In this manner, the at least one compound capable of binding to the biological target or fragment thereof can be identified from large pooled samples (that are pooled samples containing a high number r+1 of compounds comprised in the compound library provided in step a)) with high accuracy as shown in example 4. Therefore, it is understood that the method according to the first aspect of the present disclosure is highly advantageous in that it enables high-throughput screenings of large compound libraries through sensor-based assays. In that, the number of samples that need to be tested is significantly reduced as compared to a "one compound - on well" strategy.

The device

In a second aspect, the present disclosure relates to a device adapted to carry out the method according to the first aspect of the present disclosure.

According to a preferred embodiment of the present disclosure, the device adapted to carry out the method according to the first aspect of the present disclosure comprises at least one of a liquid handling system adapted to prepare the x pooled samples; and/or a sensor, wherein the sensor comprises a sensor surface having a biological target or fragment immobilized on said sensor surface; and/or a component adapted to contact each of the pooled samples with a sensor surface having a biological target or fragment immobilized on said sensor surface; and/or a determining unit adapted to determine a value indicative of a binding of one or more of the r+1 compounds in a pooled sample to the biological target or fragment thereof; and/or a unit adapted to identify the one or more compounds capable of binding to the biological target or fragment thereof by deconvolution analysis.

Definitions and general embodiments

Unless defined otherwise, all technical and scientific terms used herein have the same meaning as commonly understood by a person skilled in the art related to this invention. Also, the singular forms "a", "an", and "the" are meant to include plural reference unless it is stated otherwise.

As used herein, the terms "compound library" and "compound library provided in step a)" are used interchangeably.

As used herein, the term "fragment" refers to a compound having a molecular weight of less than 300 Da.

As used herein, the parameter "n" is the number of compounds comprised in a compound library. In other words, the parameter "n" refers to the size of the compound library.

In the context of the present disclosure, it is understood that each of the at least n compounds comprised in a compound library has a unique chemical structure. In other words, it is understood that no compound is comprised two or more times in a compound library comprising at least n compounds. In yet other words, it is understood that each of the at least n compounds comprised in a compound library has a different structure from each of the further compounds comprised in said compound library. Hence a "compound library" as used herein comprises at least n different compounds.

In the context of the present disclosure, it is understood that each of the at least n compounds is an enantiopure compound. In other words, a racemic mixture of a compound having two enantiomers is considered two compounds in the context of the present disclosure.

As used herein, the "mean molecular weight of the at least n compounds" is abbreviated as "$M_{W,n}$" and is calculated adding up the molecular weights of each of the at least n compounds ($M_W$) and dividing said sum by the number of compounds n. In other words, the mean molecular weight of the at least n compounds is calculated by the following equation:

$$M_{W,n} = \frac{\sum_1^n M_w}{n},$$

wherein n is the number of compounds, and $M_W$ is the molecular weight of each of the n compounds.

As used herein, the term "pooled sample" refers to a solution containing r+1 compounds. Therefore, the parameter "r+1" may also be referred to as the "pool size". In other words, the parameter "r+1" and the term "pool size" are used interchangeably.

A pooled sample may be prepared by mixing stock solutions of each of the r+1 compounds. In other words, preparing x pooled samples is achieved by mixing each compound of the at least n compounds comprised in the compound library with r further compounds comprised in said compound library.

In the context of the present disclosure, it is understood that the number of r+1 compounds, that is the number of compounds contained in a pooled sample, only includes compounds comprised in the compound library comprising at least n compounds. In other words, it is understood that further compounds optionally contained in a pooled sample, such as one or more protein target, one or more reporter, one or more co-factor, and/or one or more additive, do not form one of the r+1 compounds contained in a pooled sample and is not taken into account for the calculation of the number r+1.

As used herein, the parameter "r" is the number of compounds that is mixed with each of the n compounds comprised in the compound library comprising at least n compounds. Hence, the number "r+1" is representative of the number of compounds contained in a pooled sample.

In the context of the present disclosure, it is understood that each of the r+1 compounds contained in a pooled sample has a unique chemical structure. In other words, it is understood that no compound is comprised two or more times in a pooled sample. In yet other words, it is understood that each of r+1 compounds contained in a pooled sample has a different structure from each of the further compounds contained in said pooled sample. Hence, each of the pooled samples contains r+1 different compounds.

As used herein, the parameter "y" refers to the number of pooled samples containing each of the at least n compounds comprised in the compound library.

As used herein, the term "fragment thereof" refers to a fragment of the biological target. As used herein, said term and the term "fragment of a biological target" are used interchangeably. As used herein, the term "fragment of a biological target" refers to a portion or segment of a biological target. Said fragment is characterized in that the fragment retains the functional or binding properties relevant to the biological target. In other words, a "fragment of a biological target" refers to a biomolecule that exhibits the same or about the same activity in a biological assay as compared to the biological target from which said fragment originates. Therefore, it is understood that a fragment of a biological target at least comprises the amino acids forming the binding site in the biological target.

As used herein, the term "fragment of an enzyme" refers to a portion or segment of an enzyme that exhibits the same or about the same rate of converting the natural substrate of said enzyme as the enzyme itself.

As used herein, the term "fragment of a receptor" refers to a portion or segment of an receptor that exhibits the same or about the same affinity to a known ligand of said receptor as the receptor itself.

As used herein, the term "throughput" refers to the number of tested samples in a specific duration of time.

Examples

General

All reagents and instruments used in the following were purchased from commercial suppliers, unless explicitly stated otherwise.

Example 1: Providing a sensor surface having the biological target or fragment thereof immobilized on said sensor surface

An Avi-tagged/biotinylated human E3 ubiquitin ligase obtained from a commercial supplier was immobilized on a streptavidin-coated Series S Sensor Chip suitable for SPR (herein "SA-chip"; obtained from Cytiva) to provide a sensor surface having the biological target immobilized on said sensor surface.

For the immobilization, the Avi-tagged/biotinylated human E3 ubiquitin ligase was diluted to 35 $\mu$g/mL in immobilization buffer (HBS-T, 1 mM DTT, 2 mM MgCl$_2$). The SA-chip was inserted into an 8k+ Biacore instrument (obtained from Cytiva) and the system was flushed with said immobilization buffer. All reagents were prepared in a 96-well plate, according to the scheme displayed in the Biacore immobilization method:

The surface of the SA-chip was activated with an activation buffer (1 M NaCl, 50 mM NaOH in H$_2$O) for 3x60 s (flow rat was 10 $\mu$L/min). Then, the sensor surface of the SA-chip equilibrated with immobilization buffer (duration of injection: 2$\times$60 s; flow rate: 30 $\mu$L/min).

Then, the Avi-tagged/biotinylated human E3 ubiquitin ligase in immobilization buffer (at a concentration of 35 $\mu$g/mL) was immobilized on the sensor surface of the SA-chip (duration of injection: 240 s; flow rate: 10 $\mu$L/min). The unbound protein was washed from the sensor surface with washing fluid (50% isoproanlo, 0.5 mM NaCl, 25 mM NaOH) and the residual streptavidin binding sites on the sensor surface of the SA-chip were blocked with 5 $\mu$M Biocytin (duration of injection: 60 s, flow rate: 30 $\mu$L/min).

Thereby, a sensor surface having the human E3 ubiquitin ligase immobilized on said sensor surface is obtained.

Example 2: Preparing a plurality of pooled samples

A compound library containing 186,000 different compounds was created from compound libraries obtained from commercial suppliers (Enamine Ltd., WuXi AppTec, and LifeChemicals).

For a proof of concept study, 30 stock plates (each well of each plate containing a solution of one compound in DMSO) were chosen, thawed over night at room temperature (25 °C) and centrifuged at 2000 g for 20 s.

Pooling was performed using different techniques. Thereby, it was ensured that each of the compounds contained in the 30 stock plates was present in two pooled samples.

*Dipping: Creation of single use compound pools*

Each well of three 384-well plates (obtained from Greiner) were filled with 30 μL of buffer (HBS-T, 1 mM DTT, 2 mM MgCl$_2$ in H$_2$O) using Multidrop Combi+ (table-top liquid dispenser; obtained from Thermo Fisher Scientic). The 30 stock plates provided above were inserted into the left stacker of CyBi Well vario (Pipetting Robot; obtained from CyBio / Analytik Jena) and the three 384-well plates were placed in the right stacker. With the CyBi-Well vario Nanoliter Head 384 (Pin Tool), each of the compounds of each of the 10 stock plates was transferred into one well of the three assay plates in a manner resulting in pooled samples containing 10 different compounds in each well. Afterwards, the assay plates were filled up stepwise with 90 μL of buffer (HBS-T, 1 mM DTT, 2 mM MgCl$_2$ in H$_2$O), sealed with a 384-well sealing foil (obtained from Cytiva), and shaken at 1,650 rpm for 5 min.

Thus, each of the wells of the assay plates contained a final volume of 90 μL with an approximate DMSO content of 2% and 10 different compounds, each at a concentration of 10 μM. In other words, each of the wells of the assay plates contains one pooled sample containing 10 different compounds, each at a concentration of 10 μM.

*Pre-pooling: Creation of permanent compound pools*

For the pre-pooling technique, 2 μL of each of ten different compounds of the 10 stock plates provided above were transferred into one well of a compound pool plate (96- or 384- well plate; obtained from Greiner) using a liquid transfer robot (integra viaflow 96 / 384 or Biomek FXP), resulting in a total volume of 20 μL per well, containing 10 compounds at 1 mM each and 100 % DMSO, i.e., permanent compound pools.

Subsequently, assay plates are created from said compound pool plates using a liquid transfer robot (integra viaflow 96 / 384 obtained from Integra; or Biomek FXP obtained from Beckman Coulter): Each well of each assay plate was prepared with 30 μL of buffer (HBS-T, 1 mM DTT, 2 mM MgCl$_2$ in H$_2$O) and 1.8 μL of each well of eqach compound pool plate was transferred into one separate well of said assay plate. Afterwards, the assay plates were filled up stepwise with 58.2 μL of buffer (HBS-T, 1 mM DTT, 2 mM MgCl$_2$ in H$_2$O), sealed with a 384-well sealing foil (obtained from Cytiva), and shaken at 1,650 rpm for 5 min using a table-top plate shaker. Thus, each of the wells of the assay plates contained a final volume of 90 μL with an approximate DMSO content of 2% and 10 different compounds, each at a concentration of 20 μM. In other words, each of the wells of the assay plates contains one pooled sample containing 10 different compounds, each at a concentration of 20 μM.

*Acoustic dispensing - creation of single use compound pools*

Pooled samples were created from single compounds in 100% DMSO solution by acoustic transfer: 100 nL of a solution of each of ten different compounds are acoustically dispensed into one assay plate using an Echo 650 (obtained from Beckman Coulter, Brea, CA). Afterwards, each of the wells of the assay plates were filled up stepwise with 90 μL of buffer (HBS-T, 1 mM DTT, 2 mM MgCl$_2$ in H$_2$O), sealed with a 384-well sealing foil (obtained from Cytiva), and shaken at 1,650 rpm for 5 min.

Thus, each of the wells of the assay plates contained a final volume of 90 μL with an approximate DMSO content of 2% and 10 different compounds, each at a concentration of 10 μM. In other words, each of the wells of the assay plates contains one pooled sample containing 10 different compounds, each at a concentration of 10 μM.

*Preparation of auxiliary solutions (for solvent correction, blanks, and positive control)*

For the solvent correction, a DMSO dilution series with 1%, 1.52%, 2.04%, 2.56%, 3.08% and 3.6% was prepared in buffer (HBS-T, 1 mM DTT, 2 mM MgCl$_2$ in H$_2$O). The solvent correction, blanks (HBS-T, 1 mM DTT, 2 mM MgCl$_2$ in H$_2$O, 2 % DMSO) and positive control (2 μM compound in HBS-T, 1 mM DTT, 2 mM MgCl$_2$ in H$_2$O, 2 % DMSO), were pipetted into a 96-deep-well plate and sealed with a septum foil.

Example 3: Contacting each pooled sample of the pooled samples with the sensor surface and, for each pooled sample, determining a value indicative of a binding of one or more of the 10 compounds in a pooled sample to the biological target

For all experiments, an 8k+ Biacore system (SPR; obtained from Cytiva) was used.

Table 1 Protocol for determining a value indicative of a binding of one or more of the 10 compounds in a pooled sample to the biological target

| Reagent | Application | Contact time (s) | Dissociation time (s) | Flow rate ($\mu$L/min) | Purpose |
|---|---|---|---|---|---|
| Assay Buffer | 10x in the beginning | 60 | 60 | 30 | Startup, surface equilibration |
| pooled sample | Each pooled sample once | 60 | 180 | 30 | Binding of sample to immobilized protein |
| Solvent correction | Once in the beginning, every 48 cycles, once at the end | | | | Correction for deviations of DMSO content in samples/-blanks |
| Assay Buffer | Once in the beginning, every 12 cycles, once at the end | 60 | 180 | 30 | Negative control |
| Positive control | Once in the beginning, every 24 cycles, once at the end | 60 | 420 | 30 | Positive control |
| 50% DMSO | After every step | | | | Wash |

The system was flushed with assay buffer (HBS-T, 1 mM DTT, 2 mM MgCl$_2$ in H$_2$O, 2 % DMSO).

The assay plates were inserted into the instrument and the method was conducted according to the protocol shown in Table 1. Every reagent (including each of the pooled samples prepared in example 2) was injected to both flow cells of the 8k+ Biacore system ("run A").

After a run, the Biacore system was flushed with water and cleaned with *superclean,* following the standard procedure.

For each of the pooled samples, negative controls, and positive controls, a value indicative of binding of one or more of the compounds in a (pooled) sample to the biological target was determined by SPR. Said value includes a maximum response value and a time-dependent response curve.

Figure 1 shows an excerpt of a response over cycle plot of a screening run. Relative maximum binding response normalized to positive control binding is plotted versus injection cycle. Open points at ordinate value equals 100 correspond to positive controls, open points at ordinate value equals zero correspond to negative controls, and solid points represent response values of pooled samples.

Note the compound response highlighted with an arrow, serving as example in the following. Plot was created using Biacore Insight Evaluation Software Version 5.0.18.22102, with Software Extension: "Extended Screening".

Figure 2 shows the time-dependent response curve determined for the highlighted pooled sample (i.e., highlighted in figure 1). The sensorgram shows slope signal at "Analyte binding early 1". The graph was created using Biacore Insight Evaluation Software Version 5.0.18.22102, with Software Extension: "Extended Screening".

For identifying the compound(s) capable of binding to the biological target in each of the pooled samples, the whole screen was repeated using new, unique combinations of compounds contained in pooled samples, prepared according to the methods described in example 2. SPR screening measurements and evaluations were repeated in identical fashion ("run B").

Example 4: identifying the one or more compounds capable of binding to the biological target by deconvolution analysis

The values determined in example 3, i.e. maximum response values and time-dependent response curves, were evaluated on quality of control samples. Samples leading to reference binding > 10 RU were excluded. Continuous binding competence of immobilized target proteins throughout the screening run was ensured by monitoring evolution of positive control response. Likewise, potential carry-over was evaluated by inspection of negative control injections.

To be able to determine compound identity in compound pools, several report points were collected and compared in ranked order. In line with good SPR practice, lead indicator was sufficiently high maximum response value (see Fig. 1, "relative binding response late 1"), corresponding to compound induced signal increase over baseline

On secondary level, a slope signal was determined from the time-dependent response signal at a specific time point (cf. Fig. 2, point "analyte binding early 1").

For each pooled sample, lists containing these values indicative of binding of a compound to the biological target were exported from Biacore Insight Evaluation Software Version 5.0.18.22102, with Software Extension: "Extended Screening" as xlsx-files and further processed.

Full deconvolution was achieved by direct comparison of the values indicative of binding of a compound to the biological target determined in the first run ("Run A") with the values indicative of binding of a compound to the biological target determined in the second run ("Run B"), as described in the following. The screening results (maximum response values) of runs A and B are shown in Fig. 3.

All xlsx export files (obtained above) were manipulated using python packages (SciPy, version 1.9.3; pandas, version 1.5.3; openpyxl, version 3.0.10; NumPy, version 1.24.0). From report points associated with pooled samples from the first run (run A), multiple new lists now associating observed responses with individual compounds of respective pooled samples were created. Likewise, data from second run (run B), containing the same set of compounds in new, unique pooled samples (i.e., each compound in combination with nine other compounds), was manipulated identically.

At this stage, the resulting lists contain only a fraction of correct information because with high probability, observed response is caused by one of the compounds in a pool only. The true binding response contribution of individual compounds contained in the pooled samples are identified by layering correlation plots of several report points as shown in Fig. 4.

Fig. 4 shows a correlation plot of the results obtained from run A and run B. Datapoints represent maximum response values associated with individual compounds of run A (ordinate) or run B (abszissa). Linear correlation identifies compounds significantly contributing to the maximum response signal of a pooled sample. Plot was exported from visualization tool of web-based database "Collaborative Drug Discovery (CDD) vault".

In other words, a compound is identified as capable of binding to the biological target, if the maximum response value determined for two pooled samples (in runs A and B), both containing said compound, is highly similar.

If said deconvolution analysis does not allow for an unambiguous identification of the compound capable of binding to the biological target, the deconvolution may be supplemented by comparing several report points taken from the time-dependent response curves. A compound is identified as capable of binding to the biological target, if the time-dependent response curves for two pooled samples (in runs A and B), both containing said compound, is highly similar.

**Claims**

1. A method of identifying one or more compounds capable of binding to a biological target or a fragment thereof by high-throughput screening, said method comprising the steps of:

   a) Providing a compound library comprising at least n compounds, wherein n is the number of compounds comprised in said compound library;
   b) Preparing x pooled samples, wherein x is the number of pooled samples, by mixing each compound of the at least n compounds comprised in the compound library with r further compounds comprised in said compound library, wherein

      i) each compound of the at least n compounds is contained in a number y of the x pooled samples, wherein y is at least 2, and
      ii) r is at least 2;

   c) Providing a sensor surface having the biological target or fragment thereof immobilized on said sensor surface;
   d) Contacting each pooled sample of the x pooled samples with the sensor surface;
   e) For each pooled sample of the x pooled samples, determining a value indicative of a binding of one or more of the r+1 compounds in a pooled sample to the biological target or fragment thereof; and
   f) Identifying the one or more compounds capable of binding to the biological target or fragment thereof by deconvolution analysis of the values determined in step e).

2. The method according to claim 1, wherein determining the value indicative of a binding of one or more of the r+1 compounds in a pooled sample to the biological target or fragment thereof is based on surface plasmon resonance (SPR).

3. The method according to claim 1 or 2, wherein the compound library is **characterized in that** n is at least 1,000; preferably n is at least 5,000; more preferably n is at least 10,000; yet more preferably n is at least 20,000; most preferably n is at least 30,000.

4. The method according to any one of claims 1 to 3, wherein y is

   i) at least 3, and/or
   ii) 7 or less, preferably 5 or less; more preferably 4 or less.

5. The method according to any one of claims 1 to 4, wherein y is 2, or 3, or 4, or 5.

6. The method according to any one of claims 1 to 5, wherein the mean molecular weight of the at least n compounds $M_{W,n}$ is at least 320 Da, preferably the mean molecular weight of the at least n compounds $M_{W,n}$ is at least 350 Da, more preferably the mean molecular weight of the at least n compounds $M_{W,n}$ is at least 400 Da.

7. The method according to any one of claims 1 to 6, wherein r is

   i) at least 6, preferably at least 9, more preferably at least 12, more preferably at least 15; and/or
   ii) 200 or less, preferably 150 or less, more preferably 100 or less, more preferably 80 or less, more preferably 60 or less, most preferably 40 or less; or
   iii) in the range of from 6 to 200, preferably in the range of from 9 to 150, more preferably in the range of from 9 to 100, even more preferably in the range of from 12 to 80, yet more preferably in the range of from 12 to 60, yet more preferably in the range of from 12 to 40, most preferably in the range of from 15 to 40.

8. The method according to any one of claims 1 to 7, wherein step e) comprises determining a maximum response value, and/or a time-dependent response curve.

9. The method according to any one of claims 1 to 8, wherein step f) comprises a step f1) of identifying a plurality of hitpools each containing at least one compound capable of binding to the biological target or fragment thereof.

10. The method according to claim 9, wherein step f1) is performed by

    a) determining a median value from the values for each pooled sample of the x pooled samples determined in step e); and
    b) determining a median absolute deviation (MAD) from the values for each pooled sample of the x pooled samples determined in step e); wherein
    c) a hitpool is each pooled sample of the x pooled samples having a response value of at least the median response value plus w-times the median absolute deviation (MAD), wherein w is at least 2.

11. The method according to claims 9 or 10, wherein step f) is **characterized in that** the compound capable of binding to a biological target or a fragment thereof is identified if at least 75 % of the pooled samples containing said compound are hitpools, preferably at least 80 % of the pooled samples containing said compound are hitpools, more preferably at least 90 % of the pooled samples containing said compound are hitpools.

12. The method according to any one of claims 1 to 11, wherein step f) further comprises a step f3) of comparing the time-dependent response curves measured for two hitpools containing the same compound.

13. The method according to claim 12, wherein step f3) is based on a Pearson's correlation, or absolute comparison of reference points in said time-dependent response curves, or relative comparison of reference points in said time-dependent response curves.

14. A device adapted to carry out the method according to any one of claims 1 to 13.

15. The device according to claim 14 comprising

    a) a liquid handling system adapted to prepare the x pooled samples; and/or
    b) a sensor, wherein the sensor comprises a sensor surface having a biological target or fragment immobilized on said sensor surface; and/or
    c) a component adapted to contact each of the pooled samples with a sensor surface having a biological target or fragment immobilized on said sensor surface; and/or
    d) a determining unit adapted to determine a value indicative of a binding of one or more of the r+1 compounds in a pooled sample to the biological target or fragment thereof; and/or

e) a unit adapted to identify the one or more compounds capable of binding to the biological target or fragment thereof by deconvolution analysis.

Fig. 1

Fig. 2

Fig. 3

Fig. 4

**EP 4 686 943 A1**

| | Europäisches Patentamt European Patent Office Office européen des brevets | **EUROPEAN SEARCH REPORT** | **Application Number** EP 24 19 2139 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/151979 A2 (EXVIVO LABS INC [US]) 18 July 2024 (2024-07-18) * paragraphs [0020], [0048], [0056] – [0061], [0068] – [0069]; figures 3,4 * ----- | 1-15 | INV. G01N33/543 G01N21/552 |
| X | KNOWLING STUART ET AL: "Direct Comparison of Label-Free Biosensor Binding Kinetics Obtained on the Biacore 8K and the Carterra LSA", SLAS DISCOVERY: ADVANCING LIFE SCIENCES R&D, vol. 25, no. 9, 1 October 2020 (2020-10-01), pages 977-984, XP093239697, ISSN: 2472-5552, DOI: 10.1177/2472555220934814 Retrieved from the Internet: URL:https://pdf.sciencedirectassets.com/78 0758/1-s2.0-S2472555220X25093/1-s2.0-S2472 555222066382/main.pdf?hash=2a88488ac29d9cf 476258dfe6d88a691f9cadac942b8314055266262a 9e86385&host=68042c943591013ac2b2430a89b27 0f6af2c76d8dfd086a07176afe7c76c2c61&pii=S2 472555222066382&tid=spdf-e398b9de-987a-49c c-a6d1-514> * the whole document * ----- | 14,15 | |
| | -/-- | | **TECHNICAL FIELDS SEARCHED (IPC)** G01N |

The present search report has been drawn up for all claims

| Place of search | Date of completion of the search | Examiner |
|---|---|---|
| Munich | 16 January 2025 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GIANNETTI ANTHONY M: "From experimental design to validated hits a comprehensive walk-through of fragment lead identification using surface plasmon resonance", METHODS IN ENZYMOLOGY, vol. 493, 1 March 2011 (2011-03-01), pages 169-218, XP008178598, ISSN: 1557-7988, DOI: 10.1016/B978-0-12-381274-2.00008-X [retrieved on 2011-03-01] * the whole document * ----- | 1-15 | |
| A | LEI HAO ET AL: "Identification ofB. anthracisN5-carboxyaminoimidazole ribonucleotide mutase (PurE) active site binding compounds via fragment library screening", BIOORGANIC & MEDICINAL CHEMISTRY, ELSEVIER, AMSTERDAM, NL, vol. 24, no. 4, 18 December 2015 (2015-12-18), pages 596-605, XP029399986, ISSN: 0968-0896, DOI: 10.1016/J.BMC.2015.12.029 * the whole document * ----- | 1-15 | |
| | | | **TECHNICAL FIELDS SEARCHED (IPC)** |
| A | VENTON D L ET AL: "Screening combinatorial libraries", CHEMOMETRICS AND INTELLIGENT LABORATORY SYSTEMS, ELSEVIER SCIENCE PUBLISHERS B.V. AMSTERDAM, NL, vol. 48, no. 2, 2 August 1999 (1999-08-02), pages 131-150, XP004171917, ISSN: 0169-7439, DOI: 10.1016/S0169-7439(99)00009-X * the whole document * ----- | 1-15 | |

The present search report has been drawn up for all claims

| Place of search | Date of completion of the search | Examiner |
|---|---|---|
| Munich | 16 January 2025 | Jacques, Patrice |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 2139

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2025

| Patent document cited in search report | Publication date | Patent family member(s) | Publication date |
|---|---|---|---|
| WO 2024151979 A2 | 18-07-2024 | NONE | |

EPO FORM P0459

For more details about this annex : see Official Journal of the European Patent Office, No. 12/82